# EUROPÄISCHE PATENTSCHRIFT

(11) **EP 0 827 386 B1**
(45) Veröffentlichungstag und Bekanntmachung des Hinweises auf die Patenterteilung: **19.12.2001**
(21) Anmeldenummer: 96916131.4
(22) Anmeldetag: 20.05.1996
(51) Int. Cl.: A61F 2/42

(54) **ENDOPROTHESE FÜR EIN METATARSOPHALANGEALGELENK**
ENDOPROSTHESIS FOR A METATARSO-PHALANGEAL JOINT
ENDOPROTHESE POUR ARTICULATION METATARSOPHALANGIENNE

(30) Priorität: 22.05.1995 DE 19518757
(43) Veröffentlichungstag der Anmeldung: 11.03.1998
(73) Patentinhaber: PLUS ENDOPROTHETIK AG, 6343 Rotkreuz (CH)
(72) Erfinder: KNESSL, Jürg, CH-8044 Zürich (CH); SEEBER, Engelbert, D-06846 Dessau (DE)
(74) Vertreter: Popp, Eugen, Dr.
(86) Internationale Anmeldenummer: EP9602159
(87) Internationale Veröffentlichungsnummer: WO9637169

(56) Entgegenhaltungen:
- EP-A- 0 201 651
- WO-A-91/04718
- DE-A- 3 017 798
- FR-A- 2 645 735
- FR-A- 2 663 838
- FR-A- 2 709 663
- FR-A- 2 709 948
- US-A- 4 642 122
- US-A- 5 163 961
- US-A- 5 326 366

## Beschreibung

Die Erfindung betrifft eine Endoprothese für ein Metatarsophalangeal (MTP)-Gelenk, insbesondere Großzehengelenk, mit jeweils einer am proximalen Ende der Phalanx und am distalen Ende der Metatarsale, insbesondere Metatarsale 1, verankerbaren, eine konkave bzw. konvexe Ersatzgleitfläche definierenden Prothesenhälfte, wobei die Prothesenhälften jeweils lösbar am durchmessergrößeren Ende von sich konisch verjüngenden Knochenverankerungszapfen befestigbar sind und wobei die Mantelfläche der Knochenverankerungszapfen zur erhöhten Verankerung im Knochen entweder mit einem Schraubgewinde oder mit im Axialabstand voneinander ausgebildeten Umfangsrippen versehen ist.

Eine Endoprothese dieser Art ist aus der FR-A-2 709 663 bekannt. Konkret betrifft die FR-A-2 709 663 eine Endoprothese für ein MTP-Gelenk mit einer konvexen Ersatzgleitfläche, die am distalen Ende der Metatarsale verankerbar ist. Die die Ersatzgleitflächen definierende Prothesenhälfte wird an einem durchmessergrößeren Ende eines sich konisch verjüngenden Knochenverankerungszapfens befestigt. Der Knochenverankerungszapfen ist zur besseren Verankerung im Knochen mit einem Schraubgewinde versehen. Die Ersatzgleitflächen weisen eine sphärische Form auf. Eine besondere Ausgestaltung der Ersatzgleitfläche derart, daß diese zwei verschiedene Radien umfaßt, ist der FR-A-2 709 663 nicht zu entnehmen. Somit wird ein mit einer derartigen Endoprothese ausgestattetes Gelenk in seiner Bewegung abhängig von seiner Bewegungsrichtung nicht unterschiedlich durch die Form der Ersatzgleitfläche unterstützt.

Dementsprechend liegt der vorliegenden Erfindung die Aufgabe zugrunde, ausgehend von dem erwähnten Stand der Technik eine Endoprothese für ein Metatarsophalangealgelenk zu schaffen, deren Prothesenhälften eine bessere Funktionsfähigkeit des auszubildenden Gelenks gewährleisten.

Die vorgenannte Aufgabe wird erfindungsgemäß durch die Merkmale des Anspruches 1 gelöst, wobei konstruktive Details des erfindungsgemäßen Grundkonzepts in den Unteransprüchen beschrieben sind.

Der Kern der vorliegenden Erfindung liegt also darin, daß die durch die Prothesenhälften definierten Ersatzgleitflächen in Saggitalebene jeweils einen kleineren Radius aufweisen als in Richtung quer dazu bzw. in Richtung lateral/medial. Dadurch wird die Flexion/Extension des Zehengelenks nicht behindert, wohingegen das Zehengelenk in Richtung lateral/medial stabilisiert ist.

Die Ersatzgleitflächen von Phalanx und Metatarsale definierenden Prothesenhälften sind an gesonderten Knochenverankerungszapfen befestigbar, und zwar derart, daß sie bei Bedarf wieder entfernt und durch neue oder andere Prothesenhälften ersetzt werden können. Darüber hinaus ist durch die Ausbildung der erwähnten Knochenverankerungszapfen bzw. -dorne ein fester Sitz derselben sowohl in der Phalanx als auch in der Metatarsale sichergestellt, und zwar praktisch bis ultimo.

Für die Einbringung der Knochenverankerungszapfen ist die Konstruktion nach Anspruch 5 besonders vorteilhaft, wonach sich innenseitig an die axiale Ausnehmung der Knochenverankerungszapfen für die Aufnahme einer Prothesenhälfte eine weitere axiale, insbesondere durchmesserkleinere Ausnehmung anschließt, deren seitliche Begrenzungswand wenigstens einen flachen Wandabschnitt besitzt, insbesondere eine Innenvier- oder -sechskantfläche für den Angriff eines korrespondierenden Drehwerkzeuges definiert. Damit kann der Verankerungsdorn in eine entsprechende Aushöhlung der Phalanx oder Metatarsale fest eingeschraubt werden. Alternativ ist natürlich auch eine Einpressung möglich, wobei dann die Mantelfläche der Knochenverankerungszapfen vorzugsweise Umfangsrippen aufweisen. Dadurch, daß die Knochenverankerungszapfen unabhängig von den zugehörigen Prothesenhälften eingebracht werden, ist eine Beschädigung der Prothesenhälften beim Einbringen der Knochenverankerungszapfen nicht möglich. Im Gegensatz dazu besteht diese Gefahr bei einer einstückigen Ausführungsform durchaus.

Die phalangeale Prothesenhälfte ist vorzugsweise durch einen Polyethylen-, Keramik-, CrCo-Legierungs- oder Titan-Legierungs-Einsatz oder einen Einsatz aus entsprechend mechanisch belastbarem Werkstoff gebildet, der bei Bedarf oder nach Verschleiß in der beschriebenen Weise austauschbar ist. Die metatarsale Prothesenhälfte ist kugelkalottenartig ausgebildet, insbesondere mit in Draufsicht ovalem Umriß, wobei die längere Achse sich in Richtung lateral/medial erstreckt. Diese Prothesenhälfte besteht vorzugsweise aus einem knochenverträglichen Metall, insbesondere einer hochwertigen Chromlegierung, oder keramikbeschichteter Titanwerkstoff oder dergleichen abriebfestem und biokompatiblen Werkstoff.

Die metatarsale Prothesenhälfte kann bei Bedarf einen inferioren und/oder superioren Fortsatz aufweisen. Dies hängt zum einen von der tatsächlichen Beschädigung des Metatarsophalangealgelenks und zum anderen von dem Flexions-/ Extensionswinkel des zu behandelnden Zehengelenks ab.

Nachstehend werden bevorzugte Ausführungsformen der erfindungsgemäßen Endoprothese, die sowohl als Hemiprothese als auch Totalprothese ausgeführt sein kann, anhand der beigefügten Zeichnung näher erläutert. Es zeigen:
- Fig. 1: ein Metatarsophalangeal (MTP)-Gelenk in Seitenansicht;
- Fig. 2: eine Draufsicht auf ein MTP-Gelenk;
- Fig. 3: eine Vorderansicht einer mit einer Prothesenhälfte versehenen Metatarsale;
- Fig. 4: eine phalangeale Prothesenhälfte samt zugehörigem Knochenverankerungsdorn in schematischer Seitenansicht;
- Fig. 5: die phalangeale Prothesenhälfte gemäß Fig. 4 in Draufsicht;
- Fig. 6: eine andere Ausführungsform einer phalangealen Prothesenhälfte samt zugehörigem Knochenverankerungsdorn in schematischer Seitenansicht;
- Fig. 7: den Knochenverankerungsdorn gemäß Fig. 6 in Draufsicht;
- Fig. 8: eine Totalprothese für ein MTP-Gelenk in röntgenologischer Seitenansicht;
- Fig. 9: eine abgewandelte Ausführungsform einer metatarsalen Prothesenhälfte in Seitenansicht entsprechend Fig. 8; und
- Fig. 10: eine noch weitere Ausführungsform einer metatarsalen Prothesenhälfte in röntgenologischer Seitenansicht entsprechend Fig. 8.

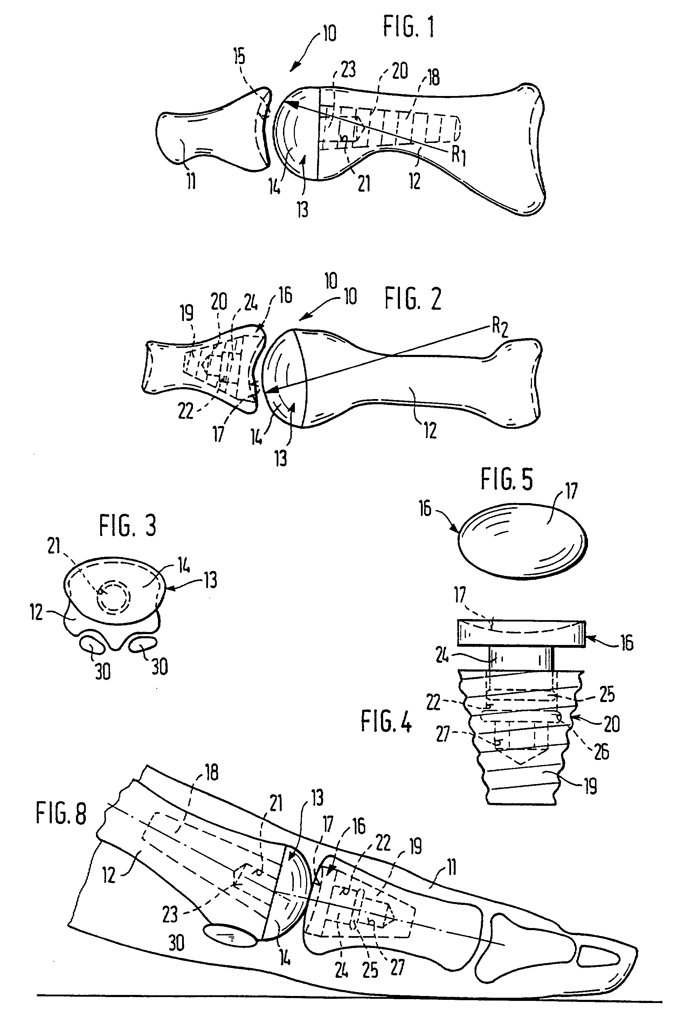

In Fig. 1 ist ein Beispiel für ein Metatarsophalangealgelenk, nämlich ein Großzehengelenk, in Draufsicht dargestellt. Mit der Bezugsziffer 11 ist die Phalanx und mit der Bezugsziffer 12 die Metatarsale I gekennzeichnet. Das Großzehengelenk ist mit der Bezugsziffer 10 angegeben. Am distalen Ende der Metatarsale 12 ist eine kugelkalottenartige Prothesenhälfte 13 unter einer entsprechenden Ersatzgleitfläche 14 angebracht, die mit der proximalen Gleitfläche 15 der Phalanx 11 zusammenwirkt. In Fig. 1 ist die Phalanx ohne Prothese dargestellt. In Fig. 2 weist die Phalanx dagegen ebenfalls eine Prothesenhälfte auf. Diese phalangeale Prothesenhälfte ist mit der Bezugsziffer 16 gekennzeichnet. Die durch diese Prothesenhälfte definierte konkave Ersatzgleitfläche weist die Bezugsziffer 17 auf. Der Radius R₁ sowohl der metatarsalen Ersatzgleitfläche 17 als auch der zugeordneten phalangealen Ersatzgleitfläche 17 ist kleiner als der Radius R₂ dieser Flächen in Richtung quer dazu bzw. in Richtung lateral/medial. Dadurch wird die Flexion/ Extension des Zehengelenks nicht behindert; andererseits wird das Zehengelenk in Richtung lateral/medial stabilisiert. In Draufsicht ist sowohl die metatarsale Ersatzgleitfläche 14 als auch die phalangeale Ersatzgleitfläche 17 etwa oval ausgebildet, wobei sich die längere Achse in Richtung lateral/medial erstreckt, so wie dies Fig. 5 sehr gut erkennen läßt.

Die erwähnten Prothesenhälften 13 und 16 sind jeweils am durchmessergrößeren Ende eines sich konisch verjüngenden Knochenverankerungszapfens 18 bzw. 19 befestigt, und zwar lösbar, wobei die Mantelfläche der Knochenverankerungszapfen 18, 19 zur erhöhten Verankerung im Knochen, hier Metatarsale 12 und Phalanx 11, entweder mit einem Schraubgewinde 20 oder mit im Axialabstand voneinander ausgebildeten Umfangsrippen versehen. Natürlich muß vor Einbringung der Knochenverankerungszapfen 18, 19 die Metatarsale 12 von distal nach proximal und die Phalanx 11 von proximal nach distal entsprechend ausgefräst werden, wobei die Fräsung so erfolgt, daß die Knochenverankerungszapfen unter radialer Vorspannung in den Knochen bzw. in die Metatarsale 12 bzw. Phalanx 11 eingebracht und dort gehalten werden. Die Knochenverankerungszapfen bestehen vorzugsweise aus einer knochenverträglichen Titanlegierung, Reintitan oder einem anderen knochenverträglichen Werkstoff mit aufgerauhter Oberfläche, wie sie für Knochenimplantate allgemein üblich ist. Damit ist ein Anwachsen des Knochens an der Oberfläche der Knochenverankerungszapfen gewährleistet. Auch sind mit knochenverträglichem Material beschichtete Verankerungskörper denkbar.

Nach Einbringen des oder der Knochenverankerungszapfen 18 bzw. 19 wird an den frei zugänglichen Enden, nämlich den jeweils durchmessergrößeren Enden die zugehörige Prothesenhälfte 13 bzw. 16 angebracht. Zu diesem Zweck weisen die Knochenverankerungszapfen 18, 19 am jeweils durchmessergrößeren, nämlich nach dem Implantieren in Knochen frei zugänglichen Ende eine axiale Ausnehmung 21 bzw. 22, vorzugsweise jeweils in Form einer Sackbohrung zur form- und/oder kraftschlüssigen Aufnahme eines zapfenartigen Befestigungsvorsprungs 23 bzw. 24 an der jeweils zugeordneten Prothesenhälfte 13 bzw. 16 auf. Die phalangeale Prothesenhälfte wird durch einen Polyethylen-, Keramik- oder Titan-Legierungs-Einsatz gebildet. Am freien Ende des zapfenartigen Befestigungsvorsprungs 24 ist bei den dargestellten Ausführungsbeispielen ein Ringvorsprung 25 ausgebildet, der mit einer entsprechenden Ringnut 26 am inneren Ende der erwähnten Ausnehmung 22 im zugeordneten Knochenverankerungszapfen 19 korrespondiert unter Ausbildung einer bei Bedarf lösbaren Rastverbindung zwischen der phalangealen Prothesenhälfte und dem zugeordneten Knochenverankerungszapfen 19. Es wird diesbezüglich insbesondere auf Fig. 4 hingewiesen.

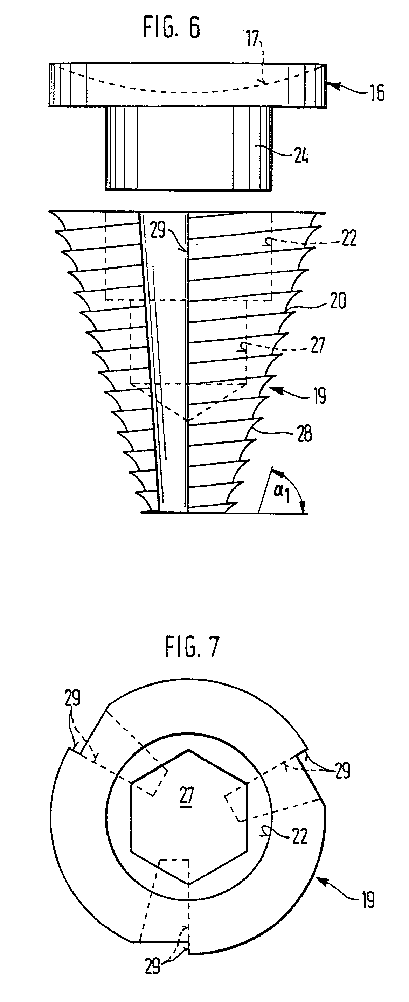

Wie insbesondere sowohl Fig. 4 als auch Fig. 6 sehr deutlich erkennen lassen, schließt sich an die axiale Ausnehmung 22 des phalangealen Knochenverankerungszapfens 19 eine weitere axiale, nämlich durchmesserkleinere Ausnehmung 27 an, deren seitliche Begrenzungswand eine Innensechskantfläche für den Angriff eines korrespondierenden Innen-Drehwerkzeuges definiert. Es wird diesbezüglich insbesondere auf die Draufsicht gemäß Fig. 7 verwiesen. In gleicher Weise kann sich auch an die axiale Ausnehmung 21 des metatarsalen Knochenverankerungszapfens 18 eine weitere Ausnehmung der vorgenannten Art anschließen.

Mit Hilfe des erwähnten Drehwerkzeuges können die Knochenverankerungszapfen fest in die zugeordneten Knochen, hier Metatarsale und Phalanx, eingedreht werden. Entsprechend Fig. 6 kann das sich über die Mantelfläche des Knochenverankerungszapfens erstreckende Schraubgewinde mit jeweils sich in Richtung zum verjüngten Ende und radial nach innen hin abfallenden Gewindeflanken 28 versehen sein, so daß die einzelnen Gewindegänge jeweils eine Art von umlaufenden Widerhaken bilden, die ein Herausziehen des Knochenverankerungszapfens aus dem zugeordneten Knochen verhindern, zumindest erheblich erschweren.

Es versteht sich von selbst, daß nicht nur phalangeale Knochenverankerungszapfen 19 entsprechend Fig. 6, sondern auch der metatarsale Knochenverankerungszapfen 18 mit dem beschriebenen Gewinde versehen sein kann.

Darüber hinaus können die Knochenverankerungszapfen noch sich axial erstreckende Knochen-Schneidkanten 29 aufweisen, so wie dies in den Fig. 6 und 7 anhand des phalangealen Knochenverankerungszapfens 19 dargestellt ist.

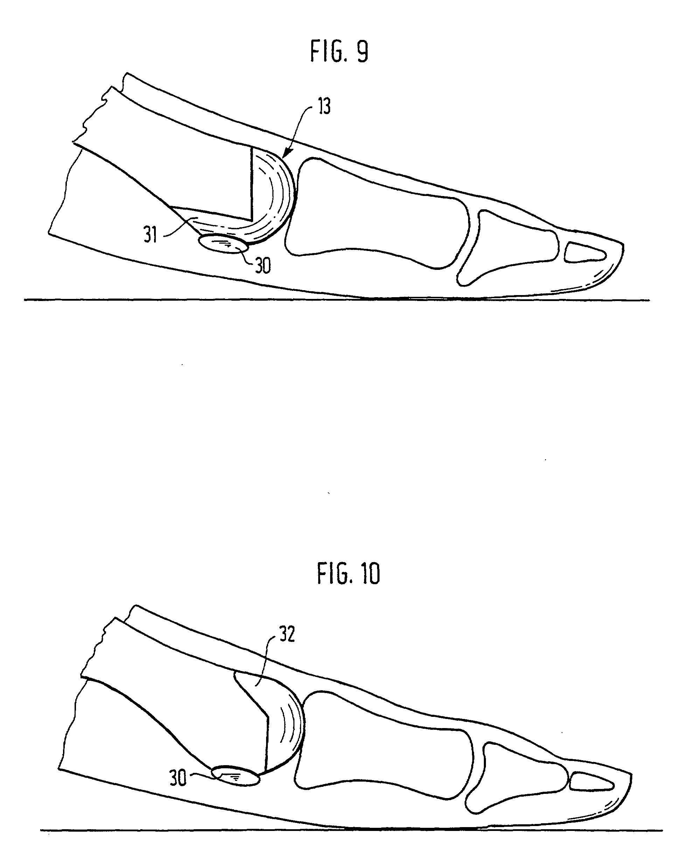

In den Fig. 3 und 8 bis 10 sind noch die der Metatarsalen 1 zugeordneten Sesambeinchen 30 dargestellt.

Des weiteren ist aus den Fig. 9 und 10 erkennbar, daß entsprechend Fig. 9 die metatarsale Prothesenhälfte 13 eine inferioren Übereck-Fortsetzung 31, und entsprechend Fig. 10 eine superiore Übereck-Fortsetzung 32 aufweisen kann. Zu der inferioren Übereck-Fortsetzung ist zu sagen, daß diese selbstverständlich an die plantare Konfiguration der Metatarsale im Distalbereich angepaßt ist, d. h. auch zur Abstützung der erwähnten Sesambeinchen 30 dient.

Die Wandung der Knochenverankerungszapfen 18, 19 kann noch radiale Durchbrüche oder Vertiefungen zur Einwachsung von Knochensubstanz aufweisen.

Es versteht sich von selbst, daß die vorbeschriebene Hemiprothese bzw. Totalprothese zementfrei implantiert wird. Des weiteren sei darauf hingewiesen, daß die beschriebene Endoprothese zwängungsfrei bzw. "non-constrained" ist.

Des weiteren sei darauf hingewiesen, daß beim Implantat für den Grundphalanx beschriebene Plateauhöhen möglich sind, wodurch man eine ausgewogene Kapselspannung wieder herstellen bzw. erzielen kann. Die Möglichkeit ist ein denbarer Teil der beschriebenen Endoprothese, der für die Praxis durchaus von Bedeutung sein kann.

### Bezugszeichenliste

- 10: Großzehengelenk
- 11: Phalanx
- 12: Metatarsale 1
- 13: Prothesenhälfte
- 14: Ersatzgleitfläche
- 15: Gleitfläche
- 16: Prothesenhälfte (phalangeal)
- 17: phalangeale Ersatzgleitfläche
- 18: Knochenverankerungszapfen (metatarsal)
- 19: Knochenverankerungszapfen (phalangeal)
- 20: Schraubgewinde
- 21: axiale Ausnehmung
- 22: axiale Ausnehmung
- 23: Befestigungsvorsprung
- 24: Befestigungsvorsprung
- 25: Ringvorsprung
- 26: Ringnut
- 27: axiale Ausnehmung
- 28: Gewindeflanken
- 29: Knochen-Schneidkante
- 30: Sesambeinchen
- 31: Übereck-Fortsatz
- 32: Übereck-Fortsatz

## Patentansprüche

1. Endoprothese für ein Metatarsophalangeal(MTP)-Gelenk, insbesondere Großzehengelenk, mit jeweils einer am proximalen Ende der Phalanx (11) und am distalen Ende der Metatarsale (12), insbesondere Metatarsale 1, verankerbaren, eine konkave (17) bzw. konvexe (14) Ersatzgleitfläche definierenden Prothesenhälfte (13, 16),
- wobei die Prothesenhälften (13, 16) jeweils lösbar am durchmessergrößeren Ende von sich konisch verjüngenden Knochenverankerungszapfen (18, 19) befestigbar sind,
- und wobei die Mantelfläche der Knochenverankerungszapfen (18, 19) zur erhöhten Verankerung im Knochen entweder mit einem Schraubgewinde (20) oder mit im Axialabstand voneinander ausgebildeten Umfangsrippen versehen ist,
**dadurch gekennzeichnet,**
**daß** die durch die Prothesenhälften (13, 16) definierten Gleitflächen (14, 17) in Sagittalebene jeweils einen kleineren Radius (R₁) aufweisen als in Richtung quer dazu bzw. in Richtung lateral/medial.

2. Endoprothese nach Anspruch 1,
**dadurch gekennzeichnet,**
**daß** bei Ausbildung von Umfangsrippen an der Mantelfläche der Knochenverankerungszapfen (18, 19) diese entweder senkrecht oder schräg zur Axialerstreckung der Zapfen verlaufen.

3. Endoprothese nach Anspruch 1 oder 2,
**dadurch gekennzeichnet,**
**daß** sowohl der in der Phalanx (11) als auch in der Metatarsalen (12) verankerbare Knochenverankerungszapfen (18, 19) am jeweils durchmessergrößeren Ende eine axiale Ausnehmung (21, 22) zur form- und/oder kraftschlüssigen Aufnahme eines zapfenartigen Befestigungsvorsprungs (23, 24) an der jeweils zugeordneten Prothesenhälfte (13, 16) aufweist.

4. Endoprothese nach Anspruch 3,
**dadurch gekennzeichnet,**
**daß** sich in den endseitig an die axiale Ausnehmung (21, 22) der Knochenverankerungszapfen (18, 19) eine weitere sich axial erstreckende, insbesondere durchmesserkleinere Ausnehmung (27) anschließt, deren seitliche Begrenzungswand wenigstens einen flachen Wandabschnitt besitzt, insbesondere eine Innenvier- oder -sechskant-fläche für den Angriff eines korrespondierenden Drehwerkzeuges definiert.

5. Endoprothese nach einem der Ansprüche 1 bis 4,
**dadurch gekennzeichnet,**
**daß** die phalangeale Prothesenhälfte (16) durch einen Polyethylen-, Keramik- oder Titan-Legierungs-Einsatz gebildet ist.

6. Endoprothese nach einem der Ansprüche 1 bis 5,
**dadurch gekennzeichnet,**
**daß** die metatarsale Prothesenhälfte (13) kugelkalottenartig ausgebildet ist, insbesondere mit in Draufsicht ovalem Umriß.

7. Endoprothese nach Anspruch 6,
**dadurch gekennzeichnet,**
**daß** die metatarsale Prothesenhälfte (13) einen inferioren und/oder superioren Übereck-Fortsatz (31; 32) aufweist.

8. Endoprothese nach einem der Ansprüche 4 bis 7,
**dadurch gekennzeichnet,**
**daß** der den Prothesenhälften (16 oder auch 13) zugeordnete zapfenartige Befestigungsvorsprung (19 oder auch 18) einen radialen Rastvorsprung (25) oder alternativ eine radiale Rastaufnahme aufweist, der mit einer komplementären Rastaufnahme (26) bzw. die mit einem komplementären Rastvorsprung in der axialen Ausnehmung (22 oder auch 21) des zugeordneten Knochenverankerungszapfens (19 oder auch 18) korrespondiert.

## Claims

1. An endoprosthesis for a metatarsophalangeal (MTP) joint, in particular the great toe joint, comprising prosthesis halves (13, 16) securable to the proximal end of the phalanx (11) and to the distal end of the metatarsal (12), in particular metatarsal 1, and defining a concave (17) and convex (14) replacement sliding surface, wherein the prosthesis halves (13, 16) are each detachably fixable to the wider end of conically tapering bone anchoring pins (18, 19), and wherein the outer surface of the bone anchoring pins (18, 19) is provided either with a screw thread (20) or with axially spaced circumferential ribs for greater anchorage in the bone, **characterised in that** the sliding surfaces (14, 17) defined by the prosthesis halves (13, 16) each have a smaller radius (R₁) in the sagittal plane than in the direction transverse thereto, i.e. the lateral/medial direction.

2. An endoprosthesis according to claim 1, **characterised in that**, if circumferential ribs are formed on the outer surface of the bone anchoring pins (18, 19), they extend either perpendicularly or obliquely to the axial extent of the pins.

3. An endoprosthesis according to claim 1 or 2, **characterised in that** the bone anchoring pins (18, 19), securable in the phalanx (11) and in the metatarsal (12), both have, at their wider end, an axial recess (21, 22) for positively and/or frictionally holding a pin-type fastening projection (23, 24) on the respective associated prosthesis half (13, 16).

4. An endoprosthesis according to claim 3, **characterised in that** an additional axially extending recess (27), in particular of smaller diameter, adjoins the end of the axial recess (21, 22) in the bone anchoring pins (18, 19), the lateral boundary wall of the recess (27) having at least one flat wall portion, in particular a square or hexagon socket surface for engagement of a corresponding turning tool.

5. An endoprosthesis according to any one of claims 1 to 4, **characterised in that** the phalangeal prosthesis half (16) is formed by a polyethylene, ceramic or titanium alloy insert.

6. An endoprosthesis according to any one of claims 1 to 5, **characterised in that** the metatarsal prosthesis half (13) is in the form of a spherical cap, in particular with an oval contour in plan view.

7. An endoprosthesis according to claim 6, **characterised in that** the metatarsal prosthesis half (13) has an inferior and/or superior diagonal extension (31, 32).

8. An endoprosthesis according to any one of claims 4 to 7, **characterised in that** the pin-type fastening projection (24, 23) associated with the prosthesis halves (16, 13) has a radial locking projection (25) or alternatively a radial locking recess, the former corresponding to a complementary locking recess (26) and the latter corresponding to a complementary locking projection in the axial recess (22, 21) in the associated bone anchoring pin (19, 18).

## Revendications

1. Endoprothèse pour une articulation métatarsophalangienne (MTP), en particulier l'articulation du gros orteil, avec à chaque fois une moitié de prothèse (13, 16) pouvant être ancrée à l'extrémité proximale de la phalange (11) et à l'extrémité distale des métatarsiens (12), en particulier du métatarsien 1, et définissant une surface de glissement de substitution concave (17), respectivement convexe (14),
- les moitiés de prothèse (13, 16) pouvant être fixées à chaque fois de manière amovible à l'extrémité de plus grand diamètre de tenons d'ancrage d'os (18, 19) coniques,
- et la surface d'enveloppe des tenons d'ancrage d'os (18, 19) étant munie, afin de renforcer l'ancrage dans l'os, soit d'un filetage (20), soit de nervures circonférentielles formées à distance axiale les unes des autres,
**caractérisée en ce que** les surfaces de glissement (14, 17) définies par les moitiés de prothèse (13, 16) présentent chacune un rayon (R₁) plus petit dans le plan sagittal que dans la direction transversale à lui ou dans la direction latérale/médiane.

2. Endoprothèse selon la revendication 1, **caractérisée en ce que**, dans le cas où des nervures circonférentielles sont formées sur la surface d'enveloppe des tenons d'ancrage d'os (18, 19), celles-ci s'étendent perpendiculairement ou à l'oblique par rapport à l'extension axiale des tenons.

3. Endoprothèse selon la revendication 1 ou 2, **caractérisée en ce que** tant le tenon d'ancrage d'os (18, 19) pouvant être ancré dans la phalange (11) que celui pouvant être ancré dans les métatarsiens (12) présente à l'extrémité de plus grand diamètre un évidement axial (21, 22) destiné à recevoir par engagement positif et/ou par adhérence une saillie de fixation (23, 24) du genre tenon située sur la moitié de prothèse (13, 16) respectivement associée.

4. Endoprothèse selon la revendication 3, **caractérisée en ce que** l'évidement axial (21, 22) du tenon d'ancrage d'os (18, 19) est suivi du côté frontal d'un autre évidement (27) d'extension axiale, en particulier de plus petit diamètre, dont la paroi limite latérale possède au moins une section de paroi plane, et définit en particulier une surface intérieure carrée ou hexagonale pour la prise d'un outil rotatif correspondant.

5. Endoprothèse selon l'une des revendications 1 à 4, **caractérisée en ce que** la moitié de prothèse phalangienne (16) est formée d'un insert en polyéthylène, en céramique ou en alliage de titane.

6. Endoprothèse selon l'une des revendications 1 à 5, **caractérisée en ce que** la moitié de prothèse métatarsienne (13) est configurée à la manière d'une calotte sphérique, en particulier avec un contour ovale en vue de dessus.

7. Endoprothèse selon la revendication 6, **caractérisée en ce que** la moitié de prothèse métatarsienne (13) présente un prolongement d'angle (31, 32) inférieur et/ou supérieur.

8. Endoprothèse selon l'une des revendications 4 à 7, **caractérisée en ce que** la saillie de fixation (19 ou aussi 18) du genre tenon associée aux moitiés de prothèse (16 ou aussi 13) présente une saillie d'encliquetage radiale (25) ou, en variante, un logement d'encliquetage radial, qui correspond avec un logement d'encliquetage complémentaire (26) ou avec une saillie d'encliquetage complémentaire dans l'évidement axial (22 ou aussi 21) du tenon d'ancrage d'os (19 ou aussi 18) associé.
